# EUROPEAN PATENT APPLICATION

(11) **EP 1 369 131 A1**
(43) Date of publication of application: **10.12.2003**
(21) Application number: 02705082.2
(22) Date of filing: 05.03.2002
(51) Int. Cl.: A61K 47/10, A61K 9/20

(54) **UTILIZATION OF SPRAY-DRIED POWDER CONTAINING SUGAR ALCOHOL**

(30) Priority: 06.03.2001 JP 2001062693
(71) Applicant: KYOWA HAKKO KOGYO CO., LTD., Chiyoda-ku, Tokyo 100-8185 (JP)
(72) Inventor: NARITA, Shoichi, c/o Kyowa Hakko Kogyo Co. Ltd., Sunto-gun, Shizuoka 411-8731 (JP); OUCHI, Kazue, c/o Kyowa Hakko Kogyo Co. Ltd., Sunto-gun, Shizuoka 411-8731 (JP); MIYABE, Junichi, c/o Kyowa Hakko Kogyo Co. Ltd., Sunto-gun, Shizuoka 411-8731 (JP); MURAI, Kouji, c/o Kyowa Hakko Kogyo Co. Ltd., Sunto-gun, Shizuoka 411-8731 (JP)
(74) Representative: Teipel, Stephan, Dr.
(86) International application number: JP0202050
(87) International publication number: WO02070013

(57) **Abstract**

In accordance with the present invention, there is provided a use of a spray-dried powder comprising a sugar alcohol for the purpose of preventing degradation or denaturation of active ingredients or changes in the functions of functional particles due to compression in manufacture of a compression-molded preparation.

## Description

### Technical Field

The present invention relates to the use of a spray-dried powder comprising a sugar alcohol for the purpose of preventing degradation or denaturation of active ingredients due to compression or for the purpose of preventing changes in functions of functional particles due to compression.

The present invention also relates to an inhibitor of degradation or denaturation of active ingredients or changes in functions of functional particles in compression-molding a preparation comprising active ingredients or functional particles to be compression-molded, which inhibitor comprises a spray-dried powder comprising a sugar alcohol.

The present invention further relates to a method of preventing degradation or denaturation of active ingredients, or for preventing changes in functions of functional particles, which is characterized in that a spray-dried powder comprising a sugar alcohol is allowed to exist therewith in compression-molding a preparation comprising active ingredients or functional particles to be compression-molded.

The present invention furthermore relates to a compression-molded preparation to prevent degradation or denaturation of active ingredients or changes in functions of functional particles, which preparation is obtained by a manufacturing method, in which a spray-dried powder comprising a sugar alcohol is allowed to exist therewith in compression-molding a preparation comprising active ingredients or functional particles to be compression-molded.

### Background of the Invention

In the field of pharmaceuticals, various pharmaceutical investigations from various aspects have been carried out concerning compression-molded preparations such as tablets. As a result, manufacture of pharmaceuticals such as tablets by compression molding has become one of the most prevailed manufacturing techniques of pharmaceutical preparations.

With regard to the compression-molded preparation, there is a demand for such a preparation that its shape is maintained and that cracking and chipping hardly occur upon handling and, therefore, improvement in hardness is desired as the aspect of the above pharmaceutical investigation concerning compression-molded preparations. However, if high hardness is merely given, it is not possible to achieve the inherent object as a pharmaceutical preparation, because degradation or denaturation of active ingredients or changes in functions of functional particles is/are caused. It is known that, although hardness usually increases when compression molding is conducted with high pressure, there are actually many cases where inconvenience in the manufacture of pharmaceutical preparations is resulted due to increased pressure for compression molding.

For example, there have been known many physiologically active ingredients which are inactivated when they are subjected to compression molding at high pressure. Further, there is a risk of breakage of particles, or breakage or detachment of coat due to the pressure upon compression molding in the case of compression-molded preparations which contain particles with a special function such as sustained release granules mentioned in Japanese Published Unexamined Patent Application No. 316042/1995 or quickly disintegrating multi-particles mentioned in Japanese Patent No. 2,820,319 or in the case of compression-molded preparations which contain coated physiologically active ingredients or particles comprising coated physiologically active ingredients for giving various preparation properties such as sustained-release, intragastrical solubility or enteric property.

Accordingly, as the art for the manufacture of compression-molded preparations, there has been a demand for a method of preventing degradation or denaturation of active ingredients due to compression or a method of preventing changes in functions of functional particles due to compression.

Those problems correspond not only to compression-molded preparations of pharmaceuticals but also compression-molded products such as tablet cakes in the field of animal drugs, foods or feed.

On the other hand, a sugar alcohol is widely used as an additive in the field of foods, pharmaceuticals or feed. From the results in actual use up to now, it has been clarified that a sugar alcohol is an additive having high safety and is excellent in chemical and biological stability. In addition, a sugar alcohol has such a characteristic that it is less apt to be a nutritive source and, therefore, it has an advantage of being used safely even when there are dietary restriction and worries about dental caries.

In recent years, preparations which rapidly disintegrate or dissolve in the oral cavity (hereinafter, referred to as intraorally rapidly disintegrable preparations ) making use of high water solubility of a sugar alcohol have been invented and actually used, and they are receiving public attention as preparations for patients having weak deglutition such as aged or infant patients.

Various studies have been carried out for making intraorally rapidly disintegrable preparations which contain a sugar alcohol into compression-molded preparations such as tablets and, as a result, there has been developed a method for the manufacture of solid preparations (such as tablets) which rapidly disintegrate in the oral cavity comprising a sugar alcohol by using special compression molding steps (Japanese Patent No. 2,919,771 and Japanese Published Unexamined Patent Application No. 12161/1999). There has been also developed an intraorally rapidly disintegrable preparation comprising a sugar alcohol which has excellent compression moldability and can be manufactured by a common compression-molding method (Japanese Published Unexamined Patent Application No. 43429/1999 and WO 97/47287).

However, in those preparations, a sugar alcohol is used to improve disintegrability of the preparations, and the use of a sugar alcohol to prevent degradation or denaturation of active ingredients due to compression or to prevent changes in functions of functional particles due to compression has not been known.

On the other hand, there is a description in Japanese Published Unexamined Patent Application No. 281564/2000 that a sugar alcohol is formulated as an additive in intraorally rapidly disintegrable tablets which contain fine particles coated with enteric coating layer. However, in the invention mentioned in the above patent, a sugar alcohol is used for obtaining sufficient strength of the preparation and sufficient disintegrability in the oral cavity, and there is no description concerning the use of a sugar alcohol to prevent degradation or denaturation of active ingredients due to compression or to prevent changes in functions of functional particles due to compression during compression molding.

In addition, the above-mentioned patent merely discloses that the intraorally rapidly disintegrable preparation of the above invention may be formulated with an additive which is commonly used in the related art and, further, there is no description about the use of a spray-dried powder of a sugar alcohol which is obtained by means of spray drying, as the sugar alcohol.

On the other hand, in a patent concerning crystalline small particles of sorbitol (Japanese Patent No. 2,874,778), a patent application concerning a method for manufacture of an excipient for direct tableting (Japanese Published Unexamined Patent Application No. 85330/1986), etc., there is a description about the use of a spray-dried powder of a sugar alcohol obtained by means of spray drying as an additive for compression-molded preparations. However, there is no description about the improvement in the instability of active ingredients due to pressure upon compression molding or about the improvement in lessening function of particles to which the function is given or particles which contain coated active ingredients or particles comprising active ingredients.

From the above, in the manufacture of compression-molded preparations, there has been no art concerning the use of a spray-dried powder comprising a sugar alcohol for the purpose of preventing degradation or denaturation of active ingredients due to compression or for the purpose of preventing changes in functions of functional particles due to compression.

### Disclosure of the Invention

An object of the present invention is to prevent degradation or denaturation of active ingredients due to compression or to prevent changes in functions of functional particles due to compression in manufacture of compression-molded preparations.

In order to accomplish the above object, the present inventors have carried out trial-and-error investigations concerning many compounds and, as a result, a sugar alcohol has been selected. That is, it has been unexpectedly found that degradation or denaturation of active ingredients or changes in functions of functional particles due to compression in manufacture of compression-molded preparations can be prevented when a spray-dried powder comprising a sugar alcohol is formulated within compression-molded preparations. On the basis of this new finding, there are provided a new use of a sugar alcohol as well as compression-molded preparations using the new use of a sugar alcohol.

The present inventors have further carried out repeated investigations and achieved the present invention.

Thus, the present invention relates to
(1) Use of a spray-dried powder comprising a sugar alcohol for preventing degradation or denaturation of active ingredients due to compression or for preventing changes in functions of functional particles due to compression in manufacture of a compression-molded preparation;
(2) The use of a spray-dried powder according to the above (1), wherein a unit particle of the spray-dried powder comprising a sugar alcohol is a primary particle;
(3) The use of a spray-dried powder according to any of the above (1) and (2), wherein an average particle size of the unit particle is 5 to 150 µm;
(4) The use of a spray-dried powder according to any of the above (1) to (3), wherein the sugar alcohol is at least one member selected from the group consisting of mannitol, xylitol, sorbitol and erythritol;
(5) The use of a spray-dried powder according to any of the above (1) to (3), wherein the sugar alcohol is D-mannitol;
(6) The use of a spray-dried powder according to any of the above (1) to (5), wherein the compression-molded preparation is a multiple-unit type tablet;
(7) The use of a spray-dried powder according to any of the above (1) to (6), wherein the compression-molded preparation is an intraorally rapidly disintegrable tablet;
(8) The use of a spray-dried powder according to any of the above (1) to (6), wherein the compression-molded preparation is a troche, a chewable tablet or a sublingual tablet; and
(9) The use of a spray-dried powder according to any of the above (1) to (8), wherein the manufacture of a compression-molded preparation is characterized in that compression molding is carried out after a lubricant is previously applied to punches and a die of a tableting machine.
   The present invention further relates to
(10) An inhibitor of degradation or denaturation of active ingredients or changes in functions of functional particles in compression-molding a preparation comprising active ingredients or functional particles to be compression-molded, which inhibitor comprises a spray-dried powder comprising a sugar alcohol;
(11) The inhibitor of degradation or denaturation of active ingredients or changes in functions of functional particles according to the above (10), wherein a unit particle of the spray-dried powder comprising a sugar alcohol is a primary particle;
(12) The inhibitor of degradation or denaturation of active ingredients or changes in functions of functional particles according to any of the above (10) and (11), wherein an average particle size of the unit particle is 5 to 150 µm;
(13) The inhibitor of degradation or denaturation of active ingredients or changes in functions of functional particles according to any of the above (10) to (12), wherein the sugar alcohol is at least one member selected from the group consisting of mannitol, xylitol, sorbitol and erythritol;
(14) The inhibitor of degradation or denaturation of active ingredients or changes in functions of functional particles according to any of the above (10) to (12), wherein the sugar alcohol is D-mannitol;
(15) The inhibitor of degradation or denaturation of active ingredients or changes in functions of functional particles according to any of the above (10) to (14), wherein the compression-molded preparation is a multiple-unit type tablet;
(16) The inhibitor of degradation or denaturation of active ingredients or changes in functions of functional particles according to any of the above (10) to (15), wherein the compression-molded preparation is a intraorally rapidly disintegrable tablet;
(17) The inhibitor of degradation or denaturation of active ingredients or changes in functions of functional particles according to any of the above (10) to (15), wherein the compression-molded preparation is a troche, a chewable tablet or a sublingual tablet; and
(18) The inhibitor of degradation or denaturation of active ingredients or changes in functions of functional particles according to any of the above (10) to (17), wherein the compression-molded preparation is a preparation obtained by compression molding after a lubricant is previously applied to punches and a die of a tableting machine.
   The present invention still further relates to
(19) A method of preventing degradation or denaturation of active ingredients or changes in functions of functional particles, which is characterized in that a spray-dried powder comprising a sugar alcohol is allowed to exist therewith in compression-molding a preparation comprising active ingredients or functional particles to be compression-molded;
(20) The method for the prevention of degradation or denaturation of active ingredients or changes in functions of functional particles according to the above (19), wherein a unit particle of the spray-dried powder comprising a sugar alcohol is a primary particle;
(21) The method for the prevention of degradation or denaturation of active ingredients or changes in functions of functional particles according to any of the above (19) and (20), wherein an average particle size of the unit particle is 5 to 150 µm;
(22) The method for the prevention of degradation or denaturation of active ingredients or changes in functions of functional particles according to any of the above (19) to (21), wherein the sugar alcohol is at least one member selected from the group consisting of mannitol, xylitol, sorbitol and erythritol;
(23) The method for the prevention of degradation or denaturation of active ingredients or changes in functions of functional particles according to any of the above (19) to (21), wherein the sugar alcohol is D-mannitol;
(24) The method for the prevention of degradation or denaturation of active ingredients or changes in functions of functional particles according to any of the above (19) to (23), wherein the compression-molded preparation is a multiple-unit type tablet;
(25) The method for the prevention of degradation or denaturation of active ingredients or changes in functions of functional particles according to any of the above (19) to (24), wherein the compression-molded preparation is a intraorally rapidly disintegrable tablet;
(26) The method for the prevention of degradation or denaturation of active ingredients or changes in functions of functional particles according to any of the above (19) to (24), wherein the compression-molded preparation is a troche, a chewable tablet or a sublingual tablet; and
(27) The method for the prevention of degradation or denaturation of active ingredients or changes in functions of functional particles according to any of the above (19) to (26), wherein the compression-molded preparation is a preparation obtained by compression molding after a lubricant is previously applied to punches and a die of a tableting machine.
   The present invention furthermore relates to
(28) A compression-molded preparation to prevent degradation or denaturation of active ingredients or changes in functions of functional particles, which preparation is obtained by a manufacturing method, in which a spray-dried powder comprising a sugar alcohol is allowed to exist therewith in compression-molding a preparation comprising active ingredients or functional particles to be compression-molded;
(29) The compression-molded preparation to prevent degradation or denaturation of active ingredients or changes in functions of functional particles according to the above (28), wherein a unit particle of the spray-dried powder comprising a sugar alcohol is a primary particle;
(30) The compression-molded preparation to prevent degradation or denaturation of active ingredients or changes in functions of functional particles according to any of the above (28) and (29), wherein an average particle size of the unit particle is 5 to 150 µm;
(31) The compression-molded preparation to prevent degradation or denaturation of active ingredients or changes in functions of functional particles according to any of the above (28) to (30), wherein the sugar alcohol is at least one member selected from the group consisting of mannitol, xylitol, sorbitol and erythritol;
(32) The compression-molded preparation to prevent degradation or denaturation of active ingredients or changes in functions of functional particles according to any of the above (28) to (30), wherein the sugar alcohol is D-mannitol;
(33) The compression-molded preparation to prevent degradation or denaturation of active ingredients or changes in functions of functional particles according to any of the above (28) to (32), wherein the compression-molded preparation is a multiple-unit type tablet;
(34) The compression-molded preparation to prevent degradation or denaturation of active ingredients or changes in functions of functional particles according to any of the above (28) to (33), wherein the compression-molded preparation is a intraorally rapidly disintegrable tablet;
(35) The compression-molded preparation to prevent degradation or denaturation of active ingredients or changes in functions of functional particles according to any of the above (28) to (33), wherein the compression-molded preparation is a troche, a chewable tablet or a sublingual tablet; and
(36) The compression-molded preparation to prevent degradation or denaturation of active ingredients or changes in functions of functional particles according to any of the above (28) to (35), wherein the compression-molded preparation is a preparation obtained by compression molding after a lubricant is previously applied to punches and a die of a tableting machine.
   The present invention still furthermore relates to
(37) The use of a spray-dried powder according to any of the above (1) to (9), wherein the manufacture of a compression-molded preparation is characterized in that compression molding is carried out by a direct tableting method;
(38) The inhibitor of degradation or denaturation of active ingredients or changes in functions of functional particles according to any of the above (10) to (18), wherein the compression-molded preparation is a preparation obtained by compression molding using a direct tableting method;
(39) The method for the prevention of degradation or denaturation of active ingredients or changes in functions of functional particles according to any of the above (19) to (27), wherein the compression-molded preparation is a preparation obtained by compression molding using a direct tableting method; and
(40) The compression-molded preparation to prevent degradation or denaturation of active ingredients or changes in functions of functional particles according to any of the above (28) to (36), wherein the compression-molded preparation is a preparation obtained by compression molding using a direct tableting method.

It is common that an ingredient which is to be administered or taken, regardless of whether it is used as a pharmaceutical or is used for other purposes such as animal drugs, foods or feed, is contained in a compression-molded preparation and, in the present invention, the ingredient is referred to as the active ingredient.

There is no particular limitation for the active ingredient to be contained in the compression-molded preparation of the present invention so far as it is a physiologically active component such as a pharmacologically active component and is for oral administration, and may be in any form such as solid, powder, crystal, oil and solution.

Its specific examples include one or more ingredient(s) selected from the group consisting of central nerve system drugs , peripheral nerve system drugs, drugs for circulation organs, drugs for respiratory organs, drugs for digestive organs, hormones, drugs for urogenital organs or anus, drugs for dental and oral use, vitamins, alimentary roborants, drugs for blood and body fluid, drugs for hepatic diseases, antidotes, drugs for habitual intoxication, gout treating agents, enzymes, antidiabetic agents, cell activation agents, anti-tumor agents, radioactive drugs, anti-allergy drugs, galenicals, antibiotics, chemotherapeutic agents, vaccines, anti-parasites drugs, diagnostic agents and narcotics/hallucinogens.

With regard to the active ingredient, it is also possible to use pharmacologically acceptable salts of the above-mentioned physiologically active ingredients, and examples thereof are, salts with inorganic acids (e.g. hydrochloric acid, sulfuric acid and nitric acid), organic acids (e.g. succinic acid, acetic acid, propionic acid and trifluoroacetic acid), inorganic bases (e.g. alkaline metals such as sodium and potassium and alkaline earth metals such as calcium and magnesium), organic basic compounds (e.g. organic amines such as triethylamine and basic amino acids such as arginine), etc.

To be more specific, examples of the physiologically active ingredients to be contained in the compression-molded preparation according to the present invention are one or more component(s) selected from the group consisting of acetylspiramycin, amoxicillin, ethyl icosapentate, itraconazole, oxatomide, glybuzole, glutathione, ketophenylbutazone, cobamamide, cisapride, todralazine, tropisetron, domperidone, valproic acid, pyridoxal, fluorouracil, flunarizine, flurazepam, benidipine, minocycline, mebendazole, medroxyprogesterone, ubidecarenone, levodopa and pharmacologically acceptable salts thereof.

Usually, in the manufacture of compression-molded preparation, the active ingredient can be decomposed or denatured as a result that the active ingredient suffers a big strain or undergoes local heating or shearing force caused by pressure during compression molding. Specific examples of degradation or denaturation of an active ingredient include reduction in the amount of the active ingredient due to degradation or denaturation as will be noted in the case where the active ingredient is pancreatin or pepsin, melting by heat as will be noted in the case where the active ingredient is ibuprofen, detachment of crystallization water or salt, transition of crystal form, and the like. Another example of degradation or denaturation of the active ingredient includes that the structure of an active ingredient forming a pore structure on the surface or an active ingredient with amorphous structure is disrupted by pressure.

With regard to the functional particle to be used in the present invention, there may be exemplified those active ingredients or particles comprising an active ingredient, which are coated with a coating agent or are made into matrix. The objects of coating or making into matrix are, for example, masking of taste and smell, making enteric or sustained-release, and stabilization including shielding of the light or exclusion of moisture. In other words, the functional particles may be those active ingredients or particles comprising an active ingredient which are coated or made into matrix by a known method, and may be in a form of fine granule or granule.

Examples of the functional particle to be used in the present invention include those particles comprising, for example, a surfactant, an absorption promoter, an organic acid or salt thereof, an absorbent or an agent for adjusting taste or smell, which are coated with a coating agent or are embedded into matrix.

Specific examples of the coating agent used in the present invention include one or more coating agent(s) selected from the group consisting of acrylic polymer (the acrylic polymer means a polymer where acrylic acid derivative(s) and/or methacrylic acid derivative(s) are polymerized) such as methacrylic acid copolymer, aminoalkyl methacrylate copolymer, carboxyvinyl polymer, etc.; cellulose polymer such as ethylcellulose, hydroxypropyl methylcellulose, hydroxypropyl cellulose, cellulose acetate phthalate, etc.; natural polymer such as acacia, pullulan, alginic acid, agar, gelatin, etc.; and fat/oil or fat/oil salt such as stearic acid, magnesium stearate, hydrogenated oil, paraffin, carnauba wax, glycerol fatty acid ester, etc. If desired, such a coating agent may be used after mixing with a plasticizer or a solubilizing agent, and examples of the plasticizer or solubilizing agent as such are polyethylene glycol, polyoxyethylene polyoxypropylene glycol, polyoxyethylene hydrogenated castor oil, triethyl citrate, polysorbate, saccharide, sugar alcohol, oligosaccharide, etc.

Further, solid dispersion or eutectic crystals manufactured from an active ingredient and other additives, particles where an active ingredient is adsorbed with porous additives, emulsion or its dried product and microcapsules or nanocapsules are also mentioned as examples of the functional particles to be used in the present invention.

Usually, in the manufacture of compression-molded preparations, functions of the above-mentioned functional particles may change due to pressure upon compression molding. A specific example of changes in function as such includes reduction in the functions such as masking, enteric property, sustained-release property or stabilization due to destruction of particles, detachment of coating agent or destruction of matrix structure. Another specific example includes separation of the drug from solid dispersion or eutectic crystals manufactured from the above active ingredient and other additives, particles where the active ingredient is adsorbed with porous additives, emulsion of dried product thereof, microcapsules or nanocapsules.

Examples of the sugar alcohol to be used in the present invention include mannitol, erythritol, xylitol, sorbitol and pyranose derivative and furanose derivative thereof, and they may be used either solely or jointly. Among them, it is preferred to use mannitol from the viewpoint of its non-hygroscopicity, high melting point, good stability, no incompatibility with an active ingredient, etc. With regard to mannitol, more preferred one is D-mannitol.

The above-mentioned spray-dried powder comprising a sugar alcohol can be easily manufactured by a spray-drying method known *per se* using a common spray-drying granulator (Spray-Drier ML-12-BS-12 manufactured by Ohkawara Kakohki is preferred) or a manufacturing machine equipped with spraying function and drying function such as a fluidized bed drying granulator, a tumbling fluidized bed granulator and a tablet coating machine. Thus, the spray-dried powder comprising a sugar alcohol is a spray-dried granulate product comprising a sugar alcohol manufactured by the above-mentioned method. Accordingly, the present invention provides, for example, (a) use of a spray-dried powder comprising a sugar alcohol for preventing degradation or denaturation of active ingredients due to compression or for preventing changes in the functions of functional particles due to compression in the manufacture of oral compression-molded preparations, (b) an inhibitor of degradation or denaturation of active ingredients or changes in the functions of functional particles in compression-molding of oral compression-molded preparations comprising active ingredients or functional particles, characterized in that, the inhibitor comprises a spray-dried powder comprising a sugar alcohol and (c) a method for preventing degradation or denaturation of active ingredients or changes in functions of functional particles, which is characterized in that a spray-dried powder comprising a sugar alcohol is allowed to exist therewith in compression-molding oral preparations comprising an active ingredient or functional particles to be compression-molded.

When a powder comprising two or more kinds of sugar alcohol is used in the compression-molded preparation of the present invention, a powder which is prepared by previously mixing the two or more sugar alcohols to make a mixed solution followed by subjecting it to spray-drying or a mixed powder which is prepared by spray-drying solutions of the two or more sugar alcohols independently followed by mixing the spray-dried powders may be used.

As for the spray-dried powder comprising a sugar alcohol used in the present invention, it is preferred that a unit particle of the spray-dried powder comprising a sugar alcohol is a primary particle. More preferably, it is suitable that an average particle size of the primary particle is about 5 to 150 µm.

The term "unit particle" means the minimum particle of a spray-dried powder when it is dispersed in liquid in which the powder is insoluble (the spray-dried powder is not dissolved therein) or floated in the air. The expression "the unit particle is a primary particle" means that the unit particle is mostly primary particles and that, preferably not less than about 70% by weight, more preferably not less than about 80% by weight or, most preferably, not less than about 90% by weight of the unit particles are primary particles. Here, the term "primary particle" is a particle where, when microscopically checked, particles are not aggregated to assemble but each particle is in an independent state. In other words, in the spray-dried powder comprising a sugar alcohol used in the present invention, the secondary particles which are formed by aggregation of the primary particles may be mixed in unit particles of the sugar alcohol.

An example of preferred methods for manufacturing the spray-dried powder comprising a sugar alcohol, of which the unit particle is a primary particle, will be illustrated below. Firstly, the sugar alcohol as mentioned above and, if necessary, other components (such as a saccharide, a binder, etc.) are completely dissolved in a solvent to prepare a solution comprising the sugar alcohol. With regard to the solvent, it is preferred to use water. Concentration of the sugar alcohol at that time is about 5 to 80% by weight or, preferably, about 10 to 45% by weight. When dissolving the sugar alcohol, it may be warmed, for example, at about 60 to 70°C.

After that, the solution of the sugar alcohol is a spray-dried by a spray-drying granulator which is known *per se* (preferably, Spray Drier ML-12-BS-12 manufactured by Ohkawara Kakohki) or by a manufacturing machine having both spraying function and drying function such as a fluidized bed drying granulator, a tumbling fluidized bed granulator or a tablet coating machine, whereupon the powder is prepared. The condition for spray-drying in the above process is endothermic temperature of about 120 to 300°C or, preferably, about 150 to 220°C.

The proportion of the active ingredient or the functional particles in the compression-molded preparation is about 0.01 to 60% by weight or, preferably, about 1 to 30% by weight.

The proportion of the spray-dried powder comprising a sugar alcohol in the compression-molded preparation is about 30 to 99.5% by weight or, preferably, about 60 to 95% by weight.

In the present invention, a spray-dried powder comprising a sugar alcohol is used for preventing degradation or denaturation of active ingredients due to compression or for preventing changes in the functions of the functional particle due to compression in the manufacture of compression-molded preparations. For example, in manufacture of a compression-molded preparation, active ingredients or functional particles, a spray-dried powder comprising a sugar alcohol and, if desired, an additive such as a disintegrant are used to manufacture the compression-molded preparation by a known method, whereupon it is possible to manufacture the compression-molded preparation in which degradation or denaturation of the active ingredients due to compression or changes in the functions of the functional particles due to compression is/are prevented.

When the spray-dried powder comprising a sugar alcohol is used as mentioned above, it can be an inhibitor of degradation or denaturation of the active ingredients or changes in the functions of functional particles in compression-molding the preparations comprising active ingredients or functional particles to be compression-molded.

A method for the prevention of degradation or denaturation of the active ingredients or changes in the functions of functional particles in accordance with the present invention can be carried out by such a manner that, the spray-dried powder comprising a sugar alcohol is allowed to exist therewith in compression-molding the preparation comprising active ingredients or functional particles to be compression-molded as described above.

The compression-molded preparation according to the present invention can be prepared from the active ingredient or the functional particles and the above-mentioned spray-dried powder comprising a sugar alcohol. The fact that types of additive are small is preferable in such respect that imbalance of bioavailability of active ingredients resulted from the additives is little and that analysis of the preparation can be carried out easily.

In the compression-molded preparation of the present invention, however, it is also possible that another disintegrant may be contained besides the above-mentioned spray-dried powder comprising a sugar alcohol.

Examples of the disintegrant to be used in the present invention include cellulose such as lowly substituted hydroxypropylcellulose and crystalline cellulose; starch or starch derivative such as corn starch, partly pregelatinized starch and hydroxypropyl starch; crospovidone; bentonite; and the like. More preferred examples include crospovidone, crystalline cellulose·carmellose sodium, sodium carboxymethyl starch or hydroxypropyl starch. The proportion of the disintegrant is preferably about 0.1 to 30% by weight or, more preferably, about 1 to 15% by weight.

It is also possible that the compression-molded preparation according to the present invention comprises other additives which are allowed to be used in preparations. To be more specific, there may be exemplified an excipient which is an inorganic salt such as calcium citrate or calcium phosphate; a lubricant such as magnesium stearate, calcium stearate, light anhydrous silicic acid or hydrated silicon dioxide; a surfactant such as sorbitan esters of fatty acid, polyoxyethylene esters of fatty acid, phospholipid, glycerol esters of fatty acid, polyethylene glycol fatty acid ester, polyoxyethylene hydrogenated castor oil, polyoxyethylene alkyl ether or sucrose esters of fatty acid; a foaming agent such as sodium bicarbonate, sodium carbonate, calcium carbonate or calcium bicarbonate; a solubilizing agent such as cyclodextrin, arginine, lysine or trisaminomethane; an organic acid such as citric acid, tartaric acid or malic acid; a color such as iron sesquioxide, yellow iron sesquioxide or tar dye; a flavor such as lemon, lemon lime, orange, pineapple, mint, menthol or camphor; a sweeten agent such as saccharin, glycyrrhizinic acid, aspartame, stevia or thaumatin; and a corrigent such as citric acid, sodium citrate, succinic acid, tartaric acid, fumaric acid or glutamic acid, and the like.

It is also possible to mix a saccharide such as lactose, sucrose, glucose or trehalose in the compression-molded preparation according to the present invention, and it is further possible to mix a binder such as hydroxypropyl cellulose, hydroxypropyl methylcellulose, pregelatinized starch, poly(vinyl alcohol), polyvinylpyrrolidone, acacia, gelatin or pullulan. Those saccharides and binders may also be mixed with a solution comprising a sugar alcohol before the solution comprising the sugar alcohol is spray-dried.

In the compression-molded preparation according to the present invention, there is no particular limitation for the proportion of the additive mentioned above but the amount may be the amount which is usually used in the related art.

Furthermore, the spray-dried powder comprising a sugar alcohol may be used for the manufacture of the compression-molded preparation after made into an additive of a premixed type being mixed with other additives or after made into an additive of a bulk type being granulated or compression-molded with other additives and they are also one of the preferred embodiments of the present invention.

With regard to the compression-molded preparation of the present invention, there is no particular limitation for its shape, use or administering method, so far as it is a compression-molded preparation comprising a preparation which can be orally administered and is manufactured by means of compression molding. The compression-molded preparation of the present invention is particularly preferred when it is a compression-molded preparation for oral administration. To be more specific, as the compression-molded preparation of the present invention, there may be exemplified buccal tablets such as internal tablets, foaming tablets, dispensing tablets, chewable tablets, troches, buccals or sublingual tablets. Other examples are sustained-release tablets such as multiple-unit type tablets, gradumets, wax matrixes, resinates or spantabs.

In the present invention, it is preferred that the spray-dried powder comprising a sugar alcohol is applied to multiple-unit type tablets, intraorally rapidly disintegrable preparations , chewable tablets, troches or sublingual tablets. Here, multiple-unit type tablets mean tablets usually prepared by mixing functional particles with powder, etc. followed by tableting, and designed in such a manner that those tablets should rapidly disintegrate upon taking them and that the functional particles move as each particle in digestive tracts. Intraorally rapidly disintegrable preparations mean preparations which are usually disintegrated or dissolved easily in the oral cavity. Chewable tablets mean tablets which are usually taken by crushing them with the teeth in the oral cavity. Troches mean tablets which are usually dissolved gradually, whereby the active ingredients are made to act on the oral cavity or the throat. Sublingual tablets mean tablets whose active ingredients are usually absorbed under the tongue to act on the whole body.

Manufacture of the compression-molded preparation of the present invention may be carried out according to a known method *per se* which is used for the manufacture of compression-molded preparations by using an active ingredient or functional particles, the above-mentioned spray-dried powder comprising a sugar alcohol and, if desired, an additive such as a disintegrant.

To be more specific, there may be exemplified an indirect tableting method where active ingredients or functional particles, the above-mentioned spray-dried powder comprising a sugar alcohol and, if desired, an additive such as a disintegrant are previously granulated by a granulator known *per se* such as fluidized bed granulation, stirring granulation, tumbling granulation, tumbling fluidized bed granulation, extrusion granulation and dry granulation, mixed with an additive such as a lubricant, and subjected to a tableting machine known *per se,* preferably a rotary tableting machine with good productivity or a single-punch tableting machine.

Another example is a direct tableting method where active ingredients or functional particles, the above-mentioned spray-dried powder comprising a sugar alcohol and, if desired, an additive such as a disintegrant are mixed and the mixture is subjected to a tableting machine known *per se,* preferably a rotary tableting machine with good productivity or a single-punch tableting machine.

In any of the above-mentioned methods, it is preferred that the pressure for compression molding is not less than about 5,000 N.

In the manufacture of the compression-molded preparation according to the present invention, any of the above-mentioned indirect tableting method and direct tableting method may be used. However the direct tableting method is easier and more convenient as compared with the indirect tableting method and, in addition, it does not cause problems in stability, etc. during granulation. Accordingly, it is preferred to adopt the direct tableting method.

In any of the manufacturing methods according to the indirect tableting method and the direct tableting method, it is possible that, during compression molding, compression molding can be carried out without mixing a lubricant with the powder in the indirect tableting method or the mixed powder in the direct tableting method by previously applying a lubricant to punch and die of the tableting machine.

### Best Mode for Carrying Out the Invention

The present invention will be further illustrated by way of the following Example and Comparative Example although they do not limit the present invention.

### Example 1

D-Mannitol (Towa Chemical Industry; an average particle size being about 60 µm) was dissolved in water to prepare a solution wherein the concentration of D-mannitol was 15% by weight. The solution was a spray-dried by a spray-drier (manufactured by Ohkawara Kakohki; Spray Drier ML-12-BS-12) to give a spray-dried powder of D-mannitol. Endothermic temperature during the spray drying was 200°C. When the spray-dried powder was observed under a microscope, it was found to be consisted of primary particles of an average particle size of about 50 µm.

The D-mannitol (1252.5 g), 75 g of crospovidone (GAF; Polyplasdone XL-10), 7.5 g of light silicic acid anhydride, 30 g of magnesium stearate and 135 g of sustained-release granules of theophylline (Eurand) were mixed. The mixture was made into tablets using a rotary tableting machine (manufactured by Hata Seisakusho; type AP-18) to give a compression-molded preparation. The tableting conditions for manufacturing were that weight per tablet was 200 mg, the metal mold was a round-shape one with sharp edge having 9 mm diameter and compression pressure was 12000 N.

### Comparative Example 1

D-Mannitol (Towa Chemical Industry; an average particle size being about 60 µm) (1252.5 g), 75 g of crospovidone (GAF; Polyplasdone XL-10), 7.5 g of light silicic acid anhydride, 30 g of magnesium stearate and 135 g of sustained-release granules of theophylline (Eurand) were mixed. The mixture was made into tablets using a rotary tableting machine (manufactured by Hata Seisakusho; type AP-18) to give the preparation. The tableting conditions were the same as those in Example 1.

### Test Example

Hardness and eluting rate of the preparations prepared in Example 1 and Comparative Example 1 were measured by the following methods. Hardness was measured by a tablet hardness tester (manufactured by Japan Machinery; type PTB-311) and average strength of ten preparations was defined as the hardness of each preparation. With regard to the eluting rate, an elution test was carried out according to the Japanese Pharmacopoeia XIII and average eluting rate at each measuring time was defined as the eluting rate for each preparation. Also, the same tests were conducted on the mixture which had not yet been subjected to tableting and was prepared in Example 1.

The result is shown in the following Table.

| Samples | Compression Pressure | Hardness | Eluting Rate | | |
|---|---|---|---|---|---|
| | | | 60 min | 180 min | 300 min |
| Before tableting | - | - | 15.8% | 42.6% | 67.0% |
| Example 1 | 12000 N | 43 N | 22.2% | 54.3% | 75.1% |
| Comp. Ex. 1 | 12000 N | Below measuring limit | 38.5% | 89.1% | 112.7% |

The preparation obtained in Example 1 using a spray-dried power comprising a sugar alcohol had hardness by which the shape was able to be maintained and, as compared with the case before tableting, changes in the eluting rate of theophylline which was an active ingredient was small. On the contrary, the preparation obtained in Comparative Example 1 using a sugar alcohol which was not obtained by spray-drying had no sufficient strength and, in addition, elution of theophylline which was an active ingredient was significantly fast as compared with the case before tableting.

The reason why elution of theophylline which is an active ingredient is significantly fast in Comparative Example 1 is probably due to degradation of functions of sustained-release granules caused by, for example, detachment of the coat. On the other hand, in Example 1, changes in the elution of theophylline which is an active ingredient as noted in Comparative Example 1 were not found, whereby it is understood that there is no change in the functions of the sustained-release granules.

### Industrial Applicability

It has become apparent in accordance with the present invention that, in the manufacture of a compression-molded preparation, a spray-dried powder comprising a sugar alcohol can be used for the purpose of preventing degradation or denaturation of the active ingredients due to compression or for the purpose of preventing changes in the functions of functional particles due to compression.

## Claims

1. Use of a spray-dried powder comprising a sugar alcohol for preventing degradation or denaturation of active ingredients due to compression or for preventing changes in functions of functional particles due to compression in manufacture of a compression-molded preparation.

2. The use of a spray-dried powder according to claim 1, wherein a unit particle of the spray-dried powder comprising a sugar alcohol is a primary particle.

3. The use of a spray-dried powder according to claim 1 or 2, wherein an average particle size of the unit particle is 5 to 150 µm.

4. The use of a spray-dried powder according to any one of claims 1 to 3, wherein the sugar alcohol is at least one member selected from the group consisting of mannitol, xylitol, sorbitol and erythritol.

5. The use of a spray-dried powder according to any one of claims 1 to 3, wherein the sugar alcohol is D-mannitol.

6. The use of a spray-dried powder according to any one of claims 1 to 5, wherein the compression-molded preparation is a multiple-unit type tablet.

7. The use of a spray-dried powder according to any one of claims 1 to 6, wherein the compression-molded preparation is an intraorally rapidly disintegrable tablet.

8. The use of a spray-dried powder according to any one of claims 1 to 6, wherein the compression-molded preparation is a troche, a chewable tablet or a sublingual tablet.

9. The use of a spray-dried powder according to any one of claims 1 to 8, wherein the manufacture of a compression-molded preparation is **characterized in that** compression molding is carried out after a lubricant is previously applied to punches and a die of a tableting machine.

10. An inhibitor of degradation or denaturation of active ingredients or changes in functions of functional particles in compression-molding a preparation comprising active ingredients or functional particles to be compression-molded, which inhibitor comprises a spray-dried powder comprising a sugar alcohol.

11. The inhibitor of degradation or denaturation of active ingredients or changes in functions of functional particles according to claim 10, wherein a unit particle of the spray-dried powder comprising a sugar alcohol is a primary particle.

12. The inhibitor of degradation or denaturation of active ingredients or changes in functions of functional particles according to claim 10 or 11, wherein an average particle size of the unit particle is 5 to 150 µm.

13. The inhibitor of degradation or denaturation of active ingredients or changes in functions of functional particles according to any one of claims 10 to 12, wherein the sugar alcohol is at least one member selected from the group consisting of mannitol, xylitol, sorbitol and erythritol.

14. The inhibitor of degradation or denaturation of active ingredients or changes in functions of functional particles according to any of claims 10 to 12, wherein the sugar alcohol is D-mannitol.

15. The inhibitor of degradation or denaturation of active ingredients or changes in functions of functional particles according to any one of claims 10 to 14, wherein the compression-molded preparation is a multiple-unit type tablet.

16. The inhibitor of degradation or denaturation of active ingredients or changes in functions of functional particles according to any one of claims 10 to 15, wherein the compression-molded preparation is an intraorally rapidly disintegrable tablet.

17. The inhibitor of degradation or denaturation of active ingredients or changes in functions of functional particles according to any one of claims 10 to 15, wherein the compression-molded preparation is a troche, a chewable tablet or a sublingual tablet.

18. The inhibitor of degradation or denaturation of active ingredients or changes in functions of functional particles according to any one of claims 10 to 17, wherein the compression-molded preparation is a preparation obtained by compression molding after a lubricant is previously applied to punches and a die of a tableting machine.

19. A method of preventing degradation or denaturation of active ingredients or changes in functions of functional particles, which is **characterized in that** a spray-dried powder comprising a sugar alcohol is allowed to exist therewith in compression-molding a preparation comprising active ingredients or functional particles to be compression-molded.

20. The method for the prevention of degradation or denaturation of active ingredients or changes in functions of functional particles according to claim 19, wherein a unit particle of the spray-dried powder comprising a sugar alcohol is a primary particle.

21. The method for the prevention of degradation or denaturation of active ingredients or changes in functions of functional particles according to claim 19 or 20, wherein an average particle size of the unit particle is 5 to 150 µm.

22. The method for the prevention of degradation or denaturation of active ingredients or changes in functions of functional particles according to any one of claims 19 to 21, wherein the sugar alcohol is at least one member selected from the group consisting of mannitol, xylitol, sorbitol and erythritol.

23. The method for the prevention of degradation or denaturation of active ingredients or changes in functions of functional particles according to any one of claims 19 to 21, wherein the sugar alcohol is D-mannitol.

24. The method for the prevention of degradation or denaturation of active ingredients or changes in functions of functional particles according to any one of claims 19 to 23, wherein the compression-molded preparation is a multiple-unit type tablet.

25. The method for the prevention of degradation or denaturation of active ingredients or changes in functions of functional particles according to any one of claims 19 to 24, wherein the compression-molded preparation is an intraorally rapidly disintegrable tablet.

26. The method for the prevention of degradation or denaturation of active ingredients or changes in functions of functional particles according to any one of claims 19 to 24, wherein the compression-molded preparation is a troche, a chewable tablet or a sublingual tablet.

27. The method for the prevention of degradation or denaturation of active ingredients or changes in functions of functional particles according to any one of claims 19 to 26, wherein the compression-molded preparation is a preparation obtained by compression molding after a lubricant is previously applied to punches and a die of a tableting machine.

28. A compression-molded preparation to prevent degradation or denaturation of active ingredients or changes in functions of functional particles, which preparation is obtained by a manufacturing method, in which a spray-dried powder comprising a sugar alcohol is allowed to exist therewith in compression-molding a preparation comprising active ingredients or functional particles to be compression-molded.

29. The compression-molded preparation to prevent degradation or denaturation of active ingredients or changes in functions of functional particles according to claim 28, wherein a unit particle of the spray-dried powder comprising a sugar alcohol is a primary particle.

30. The compression-molded preparation to prevent degradation or denaturation of active ingredients or changes in functions of functional particles according to claim 28 or 29, wherein an average particle size of the unit particle is 5 to 150 µm.

31. The compression-molded preparation to prevent degradation or denaturation of active ingredients or changes in functions of functional particles according to any one of claims 28 to 30, wherein the sugar alcohol is at least one member selected from the group consisting of mannitol, xylitol, sorbitol and erythritol.

32. The compression-molded preparation to prevent degradation or denaturation of active ingredients or changes in functions of functional particles according to any one of claims 28 to 30, wherein the sugar alcohol is D-mannitol.

33. The compression-molded preparation to prevent degradation or denaturation of active ingredients or changes in functions of functional particles according to any one of claims 28 to 32, wherein the compression-molded preparation is a multiple-unit type tablet.

34. The compression-molded preparation to prevent degradation or denaturation of active ingredients or changes in functions of functional particles according to any one of claims 28 to 33, wherein the compression-molded preparation is an intraorally rapidly disintegrable tablet.

35. The compression-molded preparation to prevent degradation or denaturation of active ingredients or changes in functions of functional particles according to any one of claims 28 to 33, wherein the compression-molded preparation is a troche, a chewable tablet or a sublingual tablet.

36. The compression-molded preparation to prevent degradation or denaturation of active ingredients or changes in functions of functional particles according to any one of claims 28 to 35, wherein the compression-molded preparation is a preparation obtained by compression molding after a lubricant is previously applied to punches and a die of a tableting machine.

37. The use of a spray-dried powder according to any one of claims 1 to 9, wherein the manufacture of a compression-molded preparation is **characterized in that** compression molding is carried out by a direct tableting method.

38. The inhibitor of degradation or denaturation of active ingredients or changes in functions of functional particles according to any one of claims 10 to 18, wherein the compression-molded preparation is a preparation obtained by compression molding using a direct tableting method.

39. The method for the prevention of degradation or denaturation of active ingredients or changes in functions of functional particles according to any one of claims 19 to 27, wherein the compression-molded preparation is a preparation obtained by compression molding using a direct tableting method.

40. The compression-molded preparation to prevent degradation or denaturation of active ingredients or changes in functions of functional particles according to any one of claims 28 to 36, wherein the compression-molded preparation is a preparation obtained by compression molding using a direct tableting method.
